# EUROPEAN PATENT SPECIFICATION

(11) **EP 4 605 368 B1**
(45) Date of publication and mention of the grant of the patent: **08.07.2026**
(21) Application number: 23832901.5
(22) Date of filing: 22.11.2023
(51) Int. Cl.: C07C 51/09, C07C 67/04, C07C 67/08, C07C 67/313

(54) **NEW METHOD FOR THE OZONOLYTIC SYNTHESIS OF HIGH MELTING DICARBOXYLIC ACIDS AND OXO-ACIDS**
NEUES VERFAHREN ZUR OZONOLYTISCHEN SYNTHESE VON HOCHSCHMELZENDEN DICARBONSÄUREN UND OXOSÄUREN
NOUVEAU PROCÉDÉ DE SYNTHÈSE OZONOLYTIQUE D'ACIDES DICARBOXYLIQUES ET D'OXO-ACIDES À POINT DE FUSION ÉLEVÉ

(30) Priority: 22.11.2022 US 202263384640 P; 06.02.2023 US 202363483405 P
(43) Date of publication of application: 27.08.2025
(73) Proprietor: P2 Science, Inc., Woodbridge, CT 06525 (US)
(72) Inventor: FOLEY, Patrick, Woodbridge, Connecticut 06525 (US); YANG, Yonghua, Woodbridge, Connecticut 06525 (US)
(74) Representative: Marks & Clerk LLP
(86) International application number: PCT/US2023/080994
(87) International publication number: WO 2024/112938

(56) References cited:
- WO-A1-2014/015290
- WO-A1-2018/053289
- CN-A- 115 784 885

## Description

### FIELD OF INVENTION

The present disclosure pertains to improved methods of preparing high melting dicarboxylic acids or oxo-acids (e.g., carboxylic acid/aldehydes) by ozonolysis of their esters. For example, the present disclosure provides methods for preparing brassylic acid by way of the ozonolysis of erucic acid ester, the method comprising the steps of converting the erucic acid to an ester, performing ozonolysis on the ester, and converting the resulting brassylate ester product to brassylic acid.

### BACKGROUND

Ozonolysis is an industrially useful transformation that involves the oxidation of an unsaturated carbon-carbon bond of an alkene using ozone. The reported mechanism (the "Criegee mechanism") begins with initial formation of a primary ozonide (1,2,3-trioxolane) intermediate which rapidly decomposes into a carbonyl compound and carbonyl oxide compound. This pair of initial intermediates recombine to form a more stable secondary ozonide (1,2,4-trioxolane), a structure featuring a peroxide bridge.

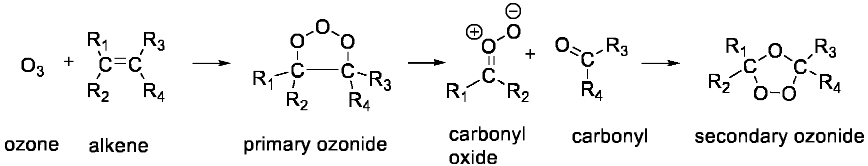

Although more stable than a primary ozonide or a carbonyl oxide, the secondary ozonide is still a high-energy chemical species subject to auto-accelerating thermal decomposition, decomposition to undesirable by-products, and organic peroxide formation (bis-peroxide, poly-peroxide, and hydroperoxide species). Therefore, further reactions must be carefully controlled in order to produce desired carbonyl product in good yield.

Traditionally, the secondary ozonide intermediates are either oxidatively or reductively cleaved to yield carbonyl products. Oxidative cleavage yields carboxylic acid and/or ketone products, while reductive cleavage yields ketone and/or aldehyde products.

The ozonolysis of numerous fatty acids and fatty acid esters have been reported. Under oxidative conditions, the product may be a diacid or a monoacid/monoester, along with a second acid, for example:

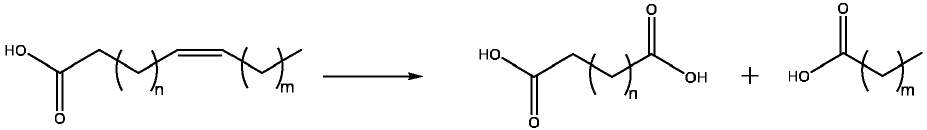

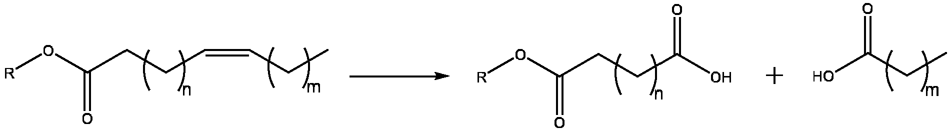

There are numerous monounsaturated fatty acids which could be subjected to such a procedure, including myristoleic acid (n=6, m=3), palmitoleic acid (n=6, m=50), sapienic acid (n=3, m=8), oleic acid (n=6, m=7), elaidic acid (n=6, m=7), vaccenic acid (n=8, m=5), and erucic acid (n=10, m=7). Simple esters of such fatty acids are also known.

Brassylic acid in particular is a very sought-after fatty acid for its use in flavor and fragrance compositions. It would be very desirable to develop a commercially-feasible, environmentally sustainable method for manufacturing brassylic acid, such as from erucic acid, which is a naturally derivable fatty acid.

The non-ozonolytic oxidative cleavage of erucic acid to form brassylic acid has been reported, e.g., using hydrogen peroxide/sodium tungsten oxide in water; hydrogen peroxide/tungstic acid in tert-butanol; sodium periodate/ruthenium trichloride in acetonitrile/ethyl acetate/water; and hydrogen peroxide in water. *See, e.g.,* Kadyrov et al., Topics in Catalysis, 57(17-20), 1366-1371 (2014); Kovash et al., ChemSusChem 7(8):2289-94 (2014); Zimmerman et al., Tetrahedron Letters 46(18):3201-03 (2005); Nieschlag, Henry et al., Industrial & Engineering Chemistry Product Research and Development 6(2):120-3 (1967); Kadyrov et al., WO 2015/036342A1; Sato et al., JP2009155302A. Such reagents, especially tungsten, iodine, and ruthenium reagents, are not environmentally friendly, though. Furthermore, hydrogen peroxide is a difficult to handle and potentially quite dangerous to work with.

The non-ozonolytic oxidative cleavage of erucic acid methyl ester to form methyl brassylate has also been reported, using a multi-step process requiring (1) formic acid/hydrogen peroxide in water, (2) sulfuric acid/tert-butanol/water, and (3) oxygen gas with a tungsten and cobalt catalyst in water. *See* Geng, Wei, et al., Huagong Jinzhan 29(7):1320-1323, 1329 (2010).

There has been one report of ozonolytic cleavage of erucic acid methyl ester using an ozone/oxygen gas mixture reacting with methyl erucate in a biphasic tert-amyl alcohol/water mixture. *See* Matthias et al., EP2573062A1 (2013). However, greater than 95% of the product mixture is nonanal and methyl 13-oxodecanonate, with only miniscule amounts of the more useful oxidative products nonanoic acid and methyl brassylate.

Batch methods for oxidative cleavage of esters are inefficient and potentially dangerous due to the collection of a large amount unstable and potentially explosive reagents and intermediates or by-products in a confined space, usually under high temperature and highpressure conditions. Ozonolysis, especially under flow conditions (e.g., in a concurrent or countercurrent flow reactor), is a particularly favorable alternative means of carrying out fatty acid or fatty ester oxidative cleavage. Such flow ozonolysis conditions combined with continuous quenching of the ozonide intermediate is particularly safe, economical, and sustainable. See, e.g., U.S. 9,035,091; 9,604,898; 10,071,944; 10,428,001; 10,934,239; 10,668,446; 10,696,605. It would be particularly advantageous to perform the continuous ozonolysis and oxidative quenching of erucic acid to form brassylic acid under neat (solvent-free) conditions, as this substantially reduces costs and complexity of the manufacturing operation. WO2014/015290A1 discloses an ozonolysis process for generation of linear alkyl aldehydes and diacids or acid-esters using elements of both, the reductive and oxidative ozonolysis of a fatty acid or a fatty acid ester, wherein the reductive and the oxidative steps are integrated in a continuous process. This document also discloses a process for preparing an oxo-acid.

However, the present inventors have unexpectedly discovered that erucic acid is unsuitable to such ozonolytic conditions. First, erucic acid is a high melting solid (molecular weight 338, melting point of about 34 °C) and manufacturing-scale process equipment must be kept at a temperature above 60 °C to reliably maintain the erucic acid in a liquid, flowable state (i.e., not too viscous). Second, when neat erucic acid is subjected to ozonolysis in a flow reactor, the product, brassylic acid (molecular weight 242, melting point about 112 °C) tends to solidify randomly, blocking the flow tubes and potentially causing overpressure and overtemperature conditions which could lead to explosions. This is all the more unexpected because the same problems are not found using the related fatty acid oleic acid (molecular weight 283, melting point 15 °C), to form azelaic acid (molecular weight 188, melting point 111 °C).

There is thus a need for an improved method of preparing brassylic acid from erucic acid, especially on a manufacturing scale. There is also a need for applying the same technology to the preparation of similar high-melting point dicarboxylic acids from their fatty acid ester precursors via ozonolysis. Such other dicarboxylic acids include azelaic acid (melting point 106 °C), undecanedioic acid (melting point 110 °C), pentadecanedioic acid (melting point > 110 °C), adipic acid (melting point 152 °C), glutaric acid (melting point 95 °C), and pimelic acid (melting point 105 °C).

### BRIEF SUMMARY OF THE INVENTION

The invention is defined in the claims.

The present disclosure provides a method for preparing a high-melting point dicarboxylic acid (e.g., brassylic acid) from a monounsaturated or polyunsaturated fatty acid (e.g., erucic acid), comprising the steps of converting the fatty acid (e.g., erucic acid) to a C₃₋₁₂ fatty acid ester (e.g., a C₃₋₁₂ erucic acid ester), performing ozonolysis with oxidative work-up on the C₃₋₁₂ fatty acid ester to form an acid/ester product (e.g., brassylic acid ester), and converting the acid ester product (e.g., brassylic acid ester) to the dicarboxylic acid (e.g., brassylic acid). In some embodiments, the ozonolysis is carried out using a flow reactor (e.g., a co-current flow reactor, such as a falling film reactor) with continuous oxidative quenching of the ozonide product stream. In some embodiments, the conversion of the acid ester to the dicarboxylic acid is conducted continuously using acid- or base-catalyzed aqueous hydrolysis.

In another embodiment, the present disclosure provides a method for preparing a high-melting point oxo-carboxylic acid (e.g., brassyl aldehyde) from a monounsaturated or polyunsaturated fatty acid (e.g., erucic acid), comprising the steps of converting the fatty acid (e.g., erucic acid) to a C₃₋₁₂ fatty acid ester (e.g., a C₃₋₁₂ erucic acid ester), performing ozonolysis with reductive work-up on the C₃₋₁₂ fatty acid ester to form an aldehyde/ester product (e.g., brassylic aldehyde ester), and converting the aldehyde ester product (e.g., brassyl aldehyde ester) to the oxo-carboxylic acid (e.g., brassyl aldehyde), wherein the ozonolysis is carried out using a flow reactor (e.g., a co-current flow reactor, such as a falling film reactor) with continuous reductive quenching of the ozonide product stream. In some embodiments, the conversion of the aldehyde ester to the oxo-carboxylic acid is conducted continuously using acid- or base-catalyzed aqueous hydrolysis.

In another embodiment, the present disclosure provides a method for preparing a high melting point dicarboxylic acid (e.g., brassylic acid) from a monounsaturated or polyunsaturated fatty acid (e.g., erucic acid), comprising the steps of converting the fatty acid to a C₃₋₁₂ fatty acid ester (e.g., a C₃₋₁₂ erucic acid ester), performing ozonolysis with reductive work-up on the C₃₋₁₂ fatty acid ester to form the oxo-carboxylic acid ester (e.g., brassylic aldehyde ester), and an aldehyde by-product (e.g., nonanal), removing the aldehyde by-product, oxidizing the oxo-carboxylic acid ester (e.g., brassylic aldehyde ester) to form the dicarboxylic acid ester (e.g., brassylic acid ester), and converting the dicarboxylic acid ester to the dicarboxylic acid (acid (e.g., brassylic acid). ). In some embodiments, the ozonolysis is carried out using a flow reactor (e.g., a co-current flow reactor, such as a falling film reactor) with continuous reductive quenching of the ozonide product stream. In some embodiments, the conversion of the acid ester to the dicarboxylic acid is conducted continuously using acid- or base-catalyzed aqueous hydrolysis.

### DETAILED DESCRIPTION OF THE INVENTION

The inventors have unexpectedly discovered that using a C₃₋₁₂ erucic acid ester as the starting material for ozonolysis results in the clean, high-yield production of brassylic acid ester, even using neat ester as the reactant, in a falling film reactor. The same benefit can similarly be obtained using other fatty acid esters to generate corresponding dicarboxylic acid products via ozonolysis, wherein said dicarboxylic acids are high melting point dicarboxylic acids.

All melting points referred to herein are melting points at standard atmospheric pressure (101,325 Pa (1 atmosphere)).

As used herein, in terms such as "C₃₋₁₂ fatty acid ester" and "C₃₋₁₂ erucic acid ester," the C₃₋₁₂" designation refers to the alcohol-derived moiety, i.e., a C₃₋₁₂ fatty acid ester is the ester product obtained from a C₃₋₁₂ alcohol condensing with a fatty acid of any chain length. Likewise, the term "C₃₋₁₂ erucic acid ester" refers to the ester product between a C₃₋₁₂ alcohol and erucic acid. Thus, in various embodiments, a C₃₋₁₂ fatty acid ester may include any ester having a linear or branched alcoholic moiety of 3-12 carbon atoms, for example, a propyl, isopropyl, n-butyl, isobutyl, sec-butyl, t-butyl, n-pentyl, isopentyl, sec-pentyl, t-pentyl, n-hexyl, n-nonyl, n-decyl, isodecyl, n-undecyl, or n-dodecyl ester. As used herein, the term "isodecyl" refers to any branched 10-carbon alkyl moiety, such as 4-dimethyloctan-2-yl, 2,6-dimethyl-octan-1-yl, 2,6-dimethyloctan-2-yl, 3,7-dimethyloctan-1-yl, and 3,7-dimethyloctan-3-yl. Isopropyl, isobutyl, and isopentyl, have their standard meanings (i.e., isopropyl is 1-methylethyl, isobutyl is 2-methylpropyl, and isopentyl is 3-methylbutyl).

It is further noted that in describing esters, the term "alcohol-derived" with references to the C₃₋₁₂ alkoxylic component of the ester equally applies where the ester is prepared synthetically using a non-alcohol mediated reaction, such as, nucleophilic condensation of the carboxylic acid with an alkene. Thus, for example, an isodecyl ester could be synthesized by reacting a fatty acid with an isodecanol or with an isodecene.

In a first embodiment of the first aspect, the present disclosure therefore provides, a method (Method 1) for preparing a high melting point dicarboxylic acid (e.g., brassylic acid) from a monounsaturated or polyunsaturated fatty acid (e.g., erucic acid), comprising the steps of:
(a) converting the fatty acid to a C₃₋₁₂ fatty acid ester;
(b) performing ozonolysis with oxidative work-up on the C₃₋₁₂ fatty acid ester to form the dicarboxylic acid ester and carboxylic acid by-product;
   and
(c) converting the dicarboxylic acid ester to the carboxylic acid product (e.g., by aqueous hydrolysis).

In further exemplary embodiments of the first aspect, the present disclosure provides:
1.1 Method 1, wherein the fatty acid is a monounsaturated fatty acid, optionally cis or trans in configuration at the double bond.
1.2 Method 1, wherein the fatty acid is a polyunsaturated fatty acid, optionally cis or trans in configuration at the double bonds.
1.3 Method 1, or any of 1.1-1.2, wherein the fatty acid has a melting point above 15 °C, e.g., above 20 °C, or above 25 °C, or above 30 °C, e.g., up to 60 °C.
1.4 Method 1, or any of 1.1-1.3, wherein the dicarboxylic acid product has a melting point above 60 °C, e.g., above 70 °C, or above 80 °C, or above 90 °C, e.g., up to 60 °C.
1.5 Method 1, or any of 1.1-1.4, wherein the fatty acid is a compound of Formula Ia or Ib:

   HOOC-(CH₂)ₙCH=CH(CH₂)ₘCH₃ Formula Ia

   HOOC-(CH₂)ₙCH=CH(CH₂CH=CH)ₚ(CH₂)ₘCH₃ Formula Ib

   wherein n is an integer from 2 to 13 (e.g., 2, 3, 4, 5, 6, 7, 9, 11, or 13) and m is an integer from 1 to 9 (e.g., 1, 4, 5, or 7), and p is an integer from 1 to 7 (e.g., 1, 2, 3, 4, or 5).
1.6 Method 1, or any of 1.1-1.5, wherein the fatty acid ester is a compound of Formula IIa or IIb:

   ROOC-(CH₂)ₙCH=CH(CH₂)mCH₃ Formula IIa

   ROOC-(CH₂)ₙCH=CH(CH₂CH=CH)ₚ(CH₂)ₘCH₃ Formula IIb

   wherein n is an integer from 2 to 13 (e.g., 2, 3, 4, 5, 6, 7, 9, 11, or 13) and m is an integer from 1 to 9 (e.g., 1, 4, 5, or 7), and p is an integer from 1 to 7 (e.g., 1, 2, 3, 4, or 5), and R is a C₃₋₁₂ alkyl (e.g., isopropyl, sec-butyl, 2-ethylhexyl, isodecyl).
1.7 Method 1, or any of 1.1-1.6, wherein the dicarboxylic acid/ester is a compound of Formula III:

   ROOC-(CH₂)ₙCOOH Formula III

   wherein n is an integer from 2 to 13 (e.g., 2, 3, 4, 5, 6, 7, 9, 11, or 13), and R is a C₃₋₁₂ alkyl (e.g., isopropyl, sec-butyl, 2-ethylhexyl, isodecyl).
1.8 Method 1, or any of 1.1-1.7, wherein the dicarboxylic acid is a compound of Formula IV:

   HOOC-(CH₂)ₙCOOH Formula IV

   wherein n is an integer from 2 to 13 (e.g., 2, 3, 4, 5, 6, 7, 9, 11, or 13).
1.9 Method 1, or any of 1.1-1.8, wherein the reaction of step (b) further provides a by-product of Formula Va or a mixture of by-products of Formulas Va and Vb:

   HOOC-(CH₂)ₘCH₃ Formula Va

   HOOCCH₂COOH Formula Vb

   wherein m is an integer from 1 to 9 (e.g., 1, 4, 5, or 7).
1.10 Method 1, or any of 1.1-1.9, wherein R is C₃₋₁₀ alkyl (e.g., isopropyl, sec-butyl, 2-ethylhexyl, isodecyl).
1.11 Method 1, or any of 1.1-1.9, wherein R is C₄₋₈ alkyl (e.g., sec-butyl, 2-ethylhexyl).
1.12 Method 1, or any of 1.1-1.9, wherein R is selected from isopropyl, n-butyl, sec-butyl, isobutyl, tert-butyl, n-pentyl, isopentyl, tert-amyl, n-hexyl, n-heptyl, 2-ethylheptyl, n-octyl, 2-ethylhexyl, n-nonyl, n-decyl, isodecyl (e.g., 2,4-dimethyloctan-2-yl, 2,6-dimethyl-octan-1-yl, 2,6-dimethyloctan-2-yl, 3,7-dimethyloctan-1-yl, and 3,7-dimethyloctan-3-yl).
1.13 Method 1, or any of 1.1-1.12, wherein the fatty acid is selected from erucic acid, palmitoleic acid, oleic acid, elaidic acid, vaccenic acid, gondonic acid, paullinic acid, nervonic acid, stearidonic acid, gamma-linolenic acid, eicosapentaenoic acid, arachidonic acid, docosatetraenonic acid, linolenic acid, linoleic acid, and linoleaidic acid.
1.14 Method 1, or any of 1.1-1.12, wherein the fatty acid is selected from erucic acid and paullinic acid.
1.15 Method 1, or any of 1.1-1.12, wherein the fatty acid is selected from palmitoleic acid, oleic acid, elaidic acid, linolenic acid, and linoleic acid.
1.16 Method 1, or any of 1.1-1.12, wherein the fatty acid is erucic acid.
1.17 Method 1, or any of 1.1-1.16, wherein the product dicarboxylic acid is selected from brassylic acid, azelaic acid, adipic acid, glutaric acid, pimelic acid, undecanedioic acid, and pentaundecanedioic acid.
1.18 Method 1, or any of 1.1-1.16, wherein the product dicarboxylic acid is selected from brassylic acid and azelaic acid.
1.19 Method 1, or any of 1.1-1.16, wherein the product is brassylic acid.
1.20 Method 1, or any of 1.1-1.19, wherein the carboxylic acid by-product(s) are selected from nonanoic acid, heptanoic acid, malonic acid, propionic acid, caproic acid, or mixtures thereof.
1.21 Method 1, or any of 1.1-1.20, wherein the step (b) ozonolysis comprises (1) treating the fatty acid ester (e.g., erucic acid ester) with gaseous ozone in a carrier gas in a flow reactor, followed by (2) continuous quenching of the flow reactor product stream under oxidative conditions.
1.22 Method 1.21, wherein the carrier gas is oxygen, nitrogen, or air.
1.23 Method 1.22, wherein the carrier gas is air (e.g., with 2.5-5% ozone in air).
1.24 Any of Methods 1.21-1.23, wherein the step (b)(1) is carried out in a flow reactor, such as a co-current flow reactor, e.g., a falling film reactor, for example, as described in U.S. 10,071,944, U.S. 10,428,001, U.S. 10,934,239, or U.S. 10,668,446.
1.25 Any of Methods 1.21-1.24, wherein the fatty acid ester (e.g., erucic acid ester) of step (a) is introduced into the reactor of step (b)(1) as an aqueous emulsion consisting of the neat fatty acid ester and aqueous solution (e.g., aqueous buffer or water), for example, without any organic solvents.
1.26 Any of Methods 1.21-1.24, wherein the fatty acid ester (e.g., erucic acid ester) of step (a) is introduced into the reactor of step (b)(1) as the neat fatty acid ester, for example, without any organic solvents or water.
1.27 Method 1.21-1.26, wherein step (b)(2) comprising continuously mixing the output stream from the reactor of step (b)(1) with a quenching solution, e.g., an aqueous solution, organic solution, or aqueous/organic emulsion, comprising an oxidizing agent.
1.28 Method 1.27, wherein the oxidizing agent is air or oxygen over a vanadium or manganese catalyst, such as vanadium oxide or manganese oxide catalyst or a vanadium or manganese complex, optionally in a carboxylic acid solvent (e.g., acetic acid).
1.29 Method 1.27, wherein the oxidizing agent is selected from hydrogen peroxide, organic peroxides (e.g., benzoyl peroxide, methyl ethyl ketone peroxide, tert-butyl hydroperoxide, peracetic acid, trifluoroperacetic acid, peroxymonosulfuric acid, meta-chloroperoxybenzoic acid, potassium monoperoxysulfate).
1.30 Method 1.21-1.29, wherein step (b) comprises water as a co-solvent, optionally, wherein the reaction of step (b) is biphasic (e.g., an emulsion).
1.31 Method 1.21-1.29, wherein step (b) is not biphasic.
1.32 Method 1.21-1.31, wherein step (b) occurs at a temperature from 30 to 100 °C.
1.33 Method 1.32, wherein said temperature is between 50 and 90 °C or between 50 and 80 °C.
1.34 Method 1, or any of 1.1-1.33, wherein the product of step (b) is purified or partially purified, e.g., by aqueous extractive work-up, by chromatography, by precipitation or crystallization, or by distillation, prior to step (c).
1.35 Method 1.34, wherein the purification provides the intermediate dicarboxylic acid ester (e.g., brassylic acid ester) in a purity of at least 80% for introduction into step (c), e.g., at least 85%, at least 90%, at least 95%, at least 97%, or at least 99% purity, e.g., as measured by HPLC or GC analysis.
1.36 Method 1.34 or 1.35, wherein the purification provides the intermediate dicarboxylic acid ester (e.g., brassylic acid ester) having not more than 20% of fatty acid or fatty acid ester (e.g., erucic acid or erucic acid ester) for introduction into step (c), e.g., not more than 15%, or not more than 10%, or not more than 5%, or not more than 3%, or not more than 2%, or not more than 1%, e.g., as measured by HPLC or GC analysis.
1.37 Method 1.34, 1.35, or 1.36, wherein the purification provides the intermediate dicarboxylic acid ester (e.g., brassylic acid ester) having not more than 20% of the carboxylic acid by-product(s) (e.g., nonanoic acid) for introduction into step (c), e.g., not more than 15%, or not more than 10%, or not more than 5%, or not more than 3%, or not more than 2%, or not more than 1%, e.g., measured by HPLC or GC analysis.
1.38 Method 1, or any of 1.1-1.37, wherein step (a) is carried out by reacting the fatty acid (e.g., erucic acid) with the corresponding C₃₋₁₂ alcohol (R-OH) or C₃₋₁₂ alkene in the presence of an acidic catalyst (e.g., a solid acidic resin).
1.39 Method 1.38, wherein step (a) is carried out by passing a mixture of the erucic acid and the C₃₋₁₂ alcohol (R-OH) or C₃₋₁₂ alkene, either neat or in a solvent, through a flow reactor comprising a solid acidic catalyst.
1.40 Method 1.38 or 1.39, wherein the acidic catalyst is an acidic polymer resin such as Amberlyst or Nafion-H, or a Montmorillonite, or a Zeolite, e.g., Montmorillonite K10, or Amberlyst H-15.
1.41 Method 1, or any of 1.1-1.40, wherein step (c) is carried out by treating the crude or purified or partially purified product from step (b) with water and an acidic or basic catalyst (e.g., aqueous sulfuric acid), optionally with a water-miscible organic solvent.
1.42 Method 1, or any of 1.1-1.40, wherein step (c) is carried out by treating the crude or purified or partially purified product from step (b) with steam under high temperature in presence of a metal or metal oxide catalyst (e.g., 150-500 °C, or 150-350 °C, or 200-400 °C, or 300-400 °C), for example, as described in U.S. Patent 6,646,146;
1.43 Method 1, or any of 1.1-1.42, wherein the product of step (c) is purified to remove reagents, unreacted starting materials and intermediates, and by-products, e.g., by aqueous extractive work-up, by chromatography, by precipitation, or crystallization, or by distillation, or any combination thereof.
1.44 Method 1.43, wherein the purification provides the dicarboxylic acid product (e.g., brassylic acid product) in a purity of at least 90%, e.g., at least 95%, at least 96%, at least 97%, at least 98%, or at least 99% purity, e.g., as measured by HPLC or GC analysis.
1.45 Method 1, or any of 1.1-1.44, wherein no step of the method comprises the use of any enzyme (e.g., any catalase enzyme).
1.46 Method 1, or any of 1.1-1.45, wherein the product stream from step (b) consists essentially of the dicarboxylic acid ester, the carboxylic acid by-product, and any unreacted fatty acid ester, or carried over fatty acid, for example, brassylic ester, nonanoic acid, and any unreacted erucic acid ester, or carried over unreacted erucic acid.
1.47 Method 1, or any of 1.1-1.46, wherein the product from step (c) consists essentially of the dicarboxylic acid product (e.g., brassylic acid).
1.48 Method 1, or any of 1.1-1.47, wherein the product stream from step (b) and/or step (c) does not contain any reduction products, e.g., the product stream does not contain any aldehydes, alcohols, aldehyde/esters, aldehyde/acids, alcohol/esters, or alcohol acids, such as those derived by reduction of any of the compounds of Formula Ia, Ib, IIa, IIb, III, IV, Va, or Vb.
1.49 Method 1.48, wherein the fatty acid is erucic acid, and the product stream from step (b) and/or step (c) does not contain any reduction products, e.g., the product stream does not contain nonanal, nonanol, 13-oxotridecanoic acid, 13-oxotridecanoate esters, 13-hydroxytridecanoic acid, or 13-hydroxytridecanoate esters.

In some embodiments according to Method 1, or any of 1.1-1.49, the fatty acid is erucic acid and the product dicarboxylic acid is brassylic acid, and the Method 1 is a method of preparing brassylic acid from erucic acid, comprising the steps of:
(a) converting the erucic acid to a C₃₋₁₂ erucic acid ester, 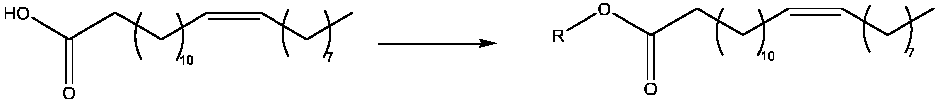 , wherein R is a C₃₋₁₂ alkyl (e.g., isopropyl, sec-butyl, 2-ethylhexyl, isodecyl);
(b) performing ozonolysis with oxidative work-up on the C₃₋₁₂ erucic acid ester to form the brassylic acid ester and nonanoic acid by-product, 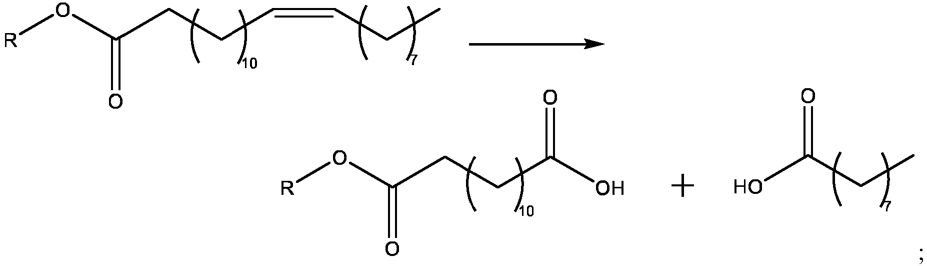 and
(c) converting the brassylic acid ester to brassylic acid (e.g., by aqueous hydrolysis)

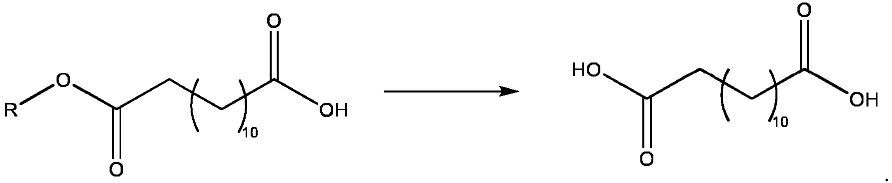

In other embodiments of Method 1, or any of 1.1-1.49, wherein the fatty acid is a different monounsaturated fatty acid, the steps of Method 1 can be drawn as follows:
(a) converting the monounsaturated fatty acid to a C₃₋₁₂ fatty acid ester, 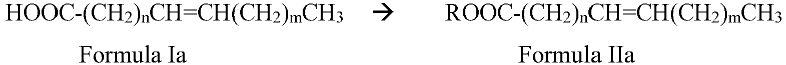 wherein n is an integer from 7 to 13 (e.g., 7, 9, 11, or 13) and m is an integer from 3 to 9 (e.g., 5 or 7), R is a C₃₋₁₂ alkyl (e.g., isopropyl, sec-butyl, 2-ethylhexyl, isodecyl);
(b) performing ozonolysis with oxidative work-up on the C₃₋₁₂ fatty acid ester to form the dicarboxylic acid ester and carboxylic acid by-product, 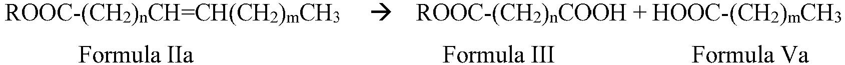 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl);
   and
(c) converting the dicarboxylic acid ester to the dicarboxylic acid product (e.g., by aqueous hydrolysis) 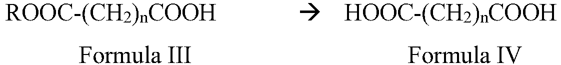 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl).

In other embodiments of Method 1, or any of 1.1-1.49, wherein the fatty acid is a polyunsaturated fatty acid, the steps of Method 1 can be drawn as follows:
(a) converting the polyunsaturated fatty acid to a C₃₋₁₂ fatty acid ester, 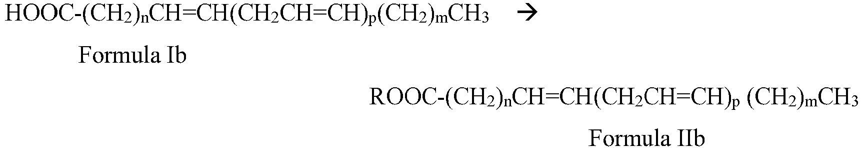 wherein n is an integer from 2 to 9 (e.g., 2, 3, 4, 5, 6, or 7) and m is an integer from 1 to 5 (e.g., 1 or 4), p is an integer from 1 to 7 (e.g., 1, 2, 3, 4, or 5), and R is a C₃₋₁₂ alkyl (e.g., isopropyl, sec-butyl, 2-ethylhexyl, isodecyl);
(b) performing ozonolysis with oxidative work-up on the C₃₋₁₂ fatty acid ester to form the dicarboxylic acid ester and carboxylic acid by-products, 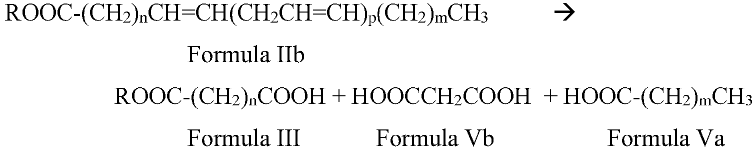 wherein n is an integer from 2 to 9 (e.g., 2, 3, 4, 5, 6, or 7) and m is an integer from 1 to 5 (e.g., 1 or 4), and R is a C₃₋₁₂ alkyl (e.g., isopropyl, sec-butyl, 2-ethylhexyl, isodecyl);
   and
(c) converting the dicarboxylic acid ester to the dicarboxylic acid product (e.g., by aqueous hydrolysis) 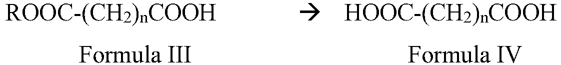 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl).

In a second embodiment of the first aspect, the present disclosure therefore provides, a method (Method 2) for preparing a high melting point oxo-carboxylic acid (e.g., brassyl aldehyde) from a monounsaturated or polyunsaturated fatty acid (e.g., erucic acid), comprising the steps of:
(a) converting the fatty acid to a C₃₋₁₂ fatty acid ester;
(b) performing ozonolysis with reductive work-up on the C₃₋₁₂ fatty acid ester to form the oxo-carboxylic acid ester and aldehyde by-product;
   and
(c) converting the oxo-carboxylic acid ester to the oxo-carboxylic acid product (e.g., by aqueous hydrolysis);
   wherein the formation of ozonide intermediate of the ozonolysis step (b) is conducted in a flow reactor.

In further exemplary embodiments of the first aspect, the present disclosure provides:
2.1.Method 2, wherein the fatty acid is a monounsaturated fatty acid, optionally cis or trans in configuration at the double bond.
2.2.Method 2, wherein the fatty acid is a polyunsaturated fatty acid, optionally cis or trans in configuration at the double bonds.
2.3.Method 2, or any of 2.1-2.2, wherein the fatty acid has a melting point above 15 °C, e.g., above 20 °C, or above 25 °C, or above 30 °C, e.g., up to 60 °C.
2.4.Method 2, or any of 2.1-2.3, wherein the dicarboxylic acid product has a melting point above 60 °C, e.g., above 70 °C, or above 80 °C, or above 90 °C, e.g., up to 60 °C.
2.5.Method 2, or any of 2.1-2.4, wherein the fatty acid is a compound of Formula VIa or VIb:

   HOOC-(CH₂)ₙCH=CH(CH₂)mCH₃ Formula VIa

   HOOC-(CH₂)ₙCH=CH(CH₂CH=CH)ₚ(CH₂)ₘCH₃ Formula VIb

   wherein n is an integer from 2 to 13 (e.g., 2, 3, 4, 5, 6, 7, 9, 11, or 13) and m is an integer from 1 to 9 (e.g., 1, 4, 5, or 7), and p is an integer from 1 to 7 (e.g., 1, 2, 3, 4, or 5).
2.6.Method 2, or any of 2.1-2.5, wherein the fatty acid ester is a compound of Formula VIIa or VIIb:

   ROOC-(CH2)ₙCH=CH(CH₂)ₘCH3 Formula VIIa

   ROOC-(CH₂)ₙCH=CH(CH₂CH=CH)ₚ(CH₂)ₘCH₃ Formula VIIb

   wherein n is an integer from 2 to 13 (e.g., 2, 3, 4, 5, 6, 7, 9, 11, or 13) and m is an integer from 1 to 9 (e.g., 1, 4, 5, or 7), and p is an integer from 1 to 7 (e.g., 1, 2, 3, 4, or 5), and R is a C₃₋₁₂ alkyl (e.g., isopropyl, sec-butyl, 2-ethylhexyl, isodecyl).
2.7.Method 2, or any of 2.1-2.6, wherein the oxo-carboxylic acid/ester is a compound of Formula VIII:

   ROOC-(CH₂)ₙCHO Formula VIII

   wherein n is an integer from 2 to 13 (e.g., 2, 3, 4, 5, 6, 7, 9, 11, or 13), and R is a C₃₋₁₂ alkyl (e.g., isopropyl, sec-butyl, 2-ethylhexyl, isodecyl).
2.8.Method 2, or any of 2.1-2.7, wherein the oxo-carboxylic acid is a compound of Formula IX:

   HOOC-(CH₂)ₙCHO Formula IX

   wherein n is an integer from 2 to 13 (e.g., 2, 3, 4, 5, 6, 7, 9, 11, or 13),
2.9. Method 2, or any of 2.1-2.8, wherein the reaction of step (b) further provides a by-product of Formula Xa or a mixture of by-products of Formulas Xa and Xb:

   OHC-(CH₂)ₘCH₃ Formula Xa

   OHCCH₂CHO Formula Xb

   wherein m is an integer from 1 to 9 (e.g., 1, 4, 5, or 7).
2.10. Method 2, or any of 2.1-2.9, wherein R is C₃₋₁₀ alkyl (e.g., isopropyl, sec-butyl, 2-ethylhexyl, isodecyl).
2.11. Method 2, or any of 2.1-2.9, wherein R is C₄₋₈ alkyl (e.g., sec-butyl, 2-ethylhexyl).
2.12. Method 2, or any of 2.1-2.9, wherein R is selected from isopropyl, n-butyl, sec-butyl, isobutyl, tert-butyl, n-pentyl, isopentyl, tert-amyl, n-hexyl, n-heptyl, 2-ethylheptyl, n-octyl, 2-ethylhexyl, n-nonyl, n-decyl, isodecyl (e.g., 2,4-dimethyloctan-2-yl, 2,6-dimethyl-octan-1-yl, 2,6-dimethyloctan-2-yl, 3,7-dimethyloctan-1-yl, and 3,7-dimethyloctan-3-yl).
2.13. Method 2, or any of 2.1-2.12, wherein the fatty acid is selected from erucic acid, palmitoleic acid, oleic acid, elaidic acid, vaccenic acid, gondonic acid, paullinic acid, nervonic acid, stearidonic acid, gamma-linolenic acid, eicosapentaenoic acid, arachidonic acid, docosatetraenonic acid, linolenic acid, linoleic acid, and linoleaidic acid.
2.14. Method 2, or any of 2.1-2.12, wherein the fatty acid is selected from erucic acid and paullinic acid.
2.15. Method 2, or any of 2.1-2.12, wherein the fatty acid is selected from palmitoleic acid, oleic acid, elaidic acid, linolenic acid, and linoleic acid.
2.16. Method 2, or any of 2.1-2.12, wherein the fatty acid is erucic acid.
2.17. Method 2, or any of 2.1-2.16, wherein the product oxo-carboxylic acid is selected from brassyl aldehyde (13-oxotridecanoic acid), 9-oxononanoic acid, 6-oxohexanoic acid, 5-oxopentanoic acid, 7-oxoheptanoic acid, 11-oxoundecanoic acid, and 15-oxopentadecanoic acid.
2.18. Method 2, or any of 2.1-2.16, wherein the product oxo-carboxylic acid is selected from brassyl aldehyde and 9-oxononanoic acid.
2.19. Method 2, or any of 2.1-2.16, wherein the product is brassyl aldehyde.
2.20. Method 2, or any of 2.1-2.19, wherein the aldehyde by-product(s) are selected from nonanal, heptanal, malonaldehyde, propanal, hexanal, or mixtures thereof.
2.21. Method 2, or any of 2.1-2.20, wherein the step (b) ozonolysis comprises (1) treating the fatty acid ester (e.g., erucic acid ester) with gaseous ozone in a carrier gas in a flow reactor, followed by (2) continuous quenching of the flow reactor product stream under reductive conditions.
2.22. Method 2.21, wherein the carrier gas is oxygen, nitrogen, or air.
2.23. Method 2.22, wherein the carrier gas is air (e.g., with 2.5-5% ozone in air).
2.24. Any of Methods 2.21-2.23, wherein the step (b)(1) is carried out in a co-current flow reactor, e.g., a falling film reactor, for example, as described in U.S. 10,071,944, U.S. 10,428,001, U.S. 10,934,239, or U.S. 10,668,446.
2.25. Any of Methods 2.21-2.24, wherein the fatty acid ester (e.g., erucic acid ester) of step (a) is introduced into the reactor of step (b)(1) as an aqueous emulsion consisting of the neat fatty acid ester and aqueous solution (e.g., aqueous buffer or water), for example, without any organic solvents.
2.26. Any of Methods 2.21-2.24, wherein the fatty acid ester (e.g., erucic acid ester) of step (a) is introduced into the reactor of step (b)(1) as the neat fatty acid ester, for example, without any organic solvents or water.
2.27. Method 2.21-2.26, wherein step (b)(2) comprising continuously mixing the output stream from the reactor of step (b)(1) with a quenching solution, e.g., an aqueous solution, organic solution, or aqueous/organic emulsion, comprising a reducing agent.
2.28. Method 2.27, wherein the reducing agent is sodium hydroxymethanesulfinate, hydroxymethanesulfinic acid, a sulfite salt (e.g., sodium sulfite, sodium bisulfite, sodium metabisulfite, potassium sulfite, potassium bisulfite, potassium metabisulfite), sulfur dioxide, sodium dithionite, and dimethyl sulfide.
2.29. Method 2.27, wherein the reducing agent is selected from triphenylphosphine, thiourea, zinc metal, hydrogen gas (e.g., over a palladium, rhodium, or platinum catalyst), or formic acid.
2.30. Method 2.27, wherein the reducing agent is glyoxal (e.g., an aqueous glyoxal solution, such as 40 wt.% aqueous glyoxal).
2.31. Method 2.21-2.30, wherein step (b) comprises water as a co-solvent, optionally, wherein the reaction of step (b) is biphasic (e.g., an emulsion).
2.32. Method 2.21-2.30, wherein step (b) is not biphasic.
2.33. Method 2.21-2.32, wherein step (b) occurs at a temperature from 30 to 100 °C.
2.34. Method 2.33, wherein said temperature is between 50 and 90 °C or between 50 and 80 °C.
2.35. Method 2, or any of 2.1-2.34, wherein the product of step (b) is purified or partially purified, e.g., by aqueous extractive work-up, by chromatography, by precipitation or crystallization, or by distillation, prior to step (c).
2.36. Method 2.35, wherein the purification provides the intermediate oxo-carboxylic acid ester (e.g., brassyl aldehyde ester) in a purity of at least 80% for introduction into step (c), e.g., at least 85%, at least 90%, at least 95%, at least 97%, or at least 99% purity, e.g., as measured by HPLC or GC analysis.
2.37. Method 2.35 or 2.36, wherein the purification provides the intermediate oxo-carboxylic acid ester (e.g., brassyl aldehyde ester) having not more than 20% of fatty acid or fatty acid ester (e.g., erucic acid or erucic acid ester) for introduction into step (c), e.g., not more than 15%, or not more than 10%, or not more than 5%, or not more than 3%, or not more than 2%, or not more than 1%, e.g., as measured by HPLC or GC analysis.
2.38. Method 2.35, 2.36, or 2.37, wherein the purification provides the intermediate oxo-carboxylic acid ester (e.g., brassyl aldehyde ester) having not more than 20% of the aldehyde by-product(s) (e.g., nonanal) for introduction into step (c), e.g., not more than 15%, or not more than 10%, or not more than 5%, or not more than 3%, or not more than 2%, or not more than 1%, e.g., as measured by HPLC or GC analysis.
2.39. Method 2, or any of 2.1-2.38, wherein step (a) is carried out by reacting the fatty acid (e.g., erucic acid) with the corresponding C₃₋₁₂ alcohol (R-OH) or C₃₋₁₂ alkene in the presence of an acidic catalyst (e.g., a solid acidic resin).
2.40. Method 2.39, wherein step (a) is carried out by passing a mixture of the erucic acid and the C₃₋₁₂ alcohol (R-OH) or C₃₋₁₂ alkene, either neat or in a solvent, through a flow reactor comprising a solid acidic catalyst.
2.41. Method 2.39 or 2.40, wherein the acidic catalyst is an acidic polymer resin such as Amberlyst or Nafion-H, or a Montmorillonite, or a Zeolite, e.g., Montmorillonite K10, or Amberlyst H-15.
2.42. Method 2, or any of 2.1-2.41, wherein step (c) is carried out by treating the crude or purified or partially purified product from step (b) with water and an acidic or basic catalyst (e.g., aqueous sulfuric acid), optionally with a water-miscible organic solvent.
2.43. Method 2, or any of 2.1-2.41, wherein step (c) is carried out by treating the crude or purified or partially purified product from step (b) with steam under high temperature in presence of a metal or metal oxide catalyst (e.g., 150-500 °C, or 150-350 °C, or 200-400 °C, or 300-400 °C), for example, as described in U.S. Patent 6,646,146;
2.44. Method 2, or any of 2.1-2.43, wherein the product of step (c) is purified to remove reagents, unreacted starting materials and intermediates, and by-products, e.g., by aqueous extractive work-up, by chromatography, by precipitation, or crystallization, or by distillation, or any combination thereof.
2.45. Method 2.44, wherein the purification provides the oxo-carboxylic acid product (e.g., brassyl aldehyde product) in a purity of at least 90%, e.g., at least 95%, at least 96%, at least 97%, at least 98%, or at least 99% purity, e.g., as measured by HPLC or GC analysis.
2.46. Method 2, or any of 2.1-2.45, wherein no step of the method comprises the use of any enzyme (e.g., any catalase enzyme).
2.47. Method 2, or any of 2.1-2.46, wherein the product stream from step (b) consists essentially of the oxo-carboxylic acid ester, the aldehyde by-product, and any unreacted fatty acid ester, or carried over fatty acid, for example, brassyl aldehyde ester, nonanal, and any unreacted erucic acid ester, or carried over unreacted erucic acid.
2.48. Method 2, or any of 2.1-2.47, wherein the product from step (c) consists essentially of the oxo-carboxylic acid product (e.g., brassyl aldehyde).
2.49. Method 2, or any of 2.1-2.48, wherein the product stream from step (b) and/or step (c) does not contain any oxidation products, e.g., the product stream does not contain any carboxylic acids, carboxylic esters, or carboxylic acid/esters, such as those derived by oxidation of any of the compounds of Formula VIa, VIb, VIIa, VIIb, VIII, IX, Xa, or Xb.
2.50. Method 2.49, wherein the fatty acid is erucic acid, and the product stream from step (b) and/or step (c) does not contain any oxidation products, e.g., the product stream does not contain nonanoic acid, brassylic acid, nonanoic acid esters or brassylic acid esters.
2.51. Method 2, or any of 2.1-2.50, wherein the method further comprises the step of isolating the aldehyde by-product (e.g., nonanal) from the crude product, or partially purified product, of step (b) or of step (c).
2.52. Method 2, or any of 2.1-2.50, wherein the step of purifying or partially purifying the crude product of step (b) or step (c) comprises the isolation of the aldehyde by-product (e.g., nonanal).
2.53. Method 2.51 or 2.52, where the aldehyde by-product (e.g., nonanal) is obtained by distillation.

In some embodiments according to Method 2, or any of 2.1-2.53, the fatty acid is erucic acid and the product oxo-carboxylic acid is brassyl aldehyde, and the Method 2 is a method of preparing brassyl aldehyde from erucic acid, comprising the steps of:
(a) converting the erucic acid to a C₃₋₁₂ erucic acid ester, 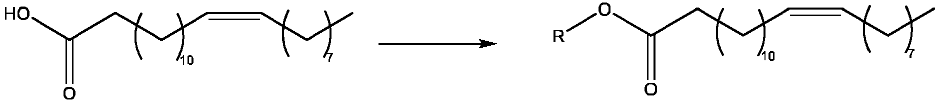 , wherein R is a C₃₋₁₂ alkyl (e.g., isopropyl, sec-butyl, 2-ethylhexyl, isodecyl);
(b) performing ozonolysis with reductive work-up on the C₃₋₁₂ erucic acid ester to form the brassyl aldehyde ester and nonanal by-product, 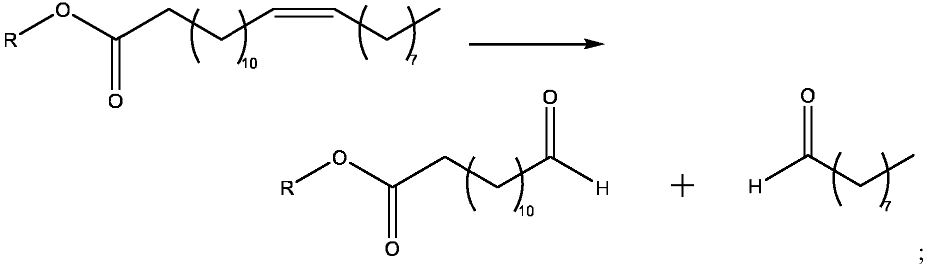 and
(c) converting the brassyl aldehyde ester to brassyl aldehyde (e.g., by aqueous hydrolysis) 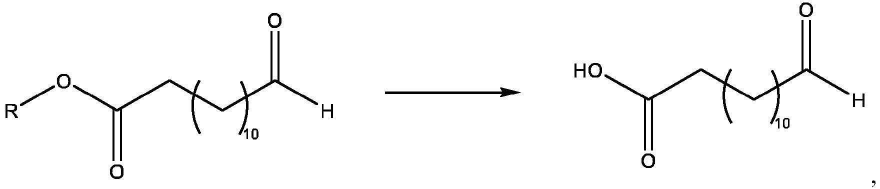 optionally further comprising the isolation and purification of the nonanal by-product.

In other embodiments of Method 2, or any of 2.1-2.53, wherein the fatty acid is a different monounsaturated fatty acid, the steps of Method 2 can be drawn as follows:
(a) converting the monounsaturated fatty acid to a C₃₋₁₂ fatty acid ester, 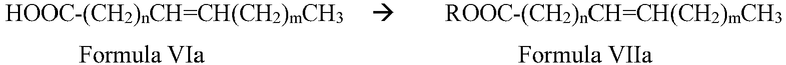 wherein n is an integer from 7 to 13 (e.g., 7, 9, 11, or 13) and m is an integer from 3 to 9 (e.g., 5 or 7), R is a C₃₋₁₂ alkyl (e.g., isopropyl, sec-butyl, 2-ethylhexyl, isodecyl);
(b) performing ozonolysis with reductive work-up on the C₃₋₁₂ fatty acid ester to form the oxo-carboxylic acid ester and aldehyde by-product, 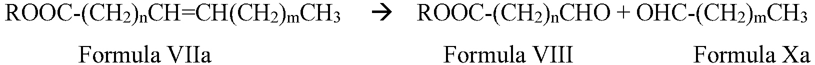 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl);
   and
(c) converting the oxo-carboxylic acid ester to the oxo-carboxylic acid product (e.g., by aqueous hydrolysis) 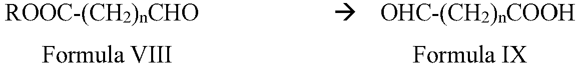 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl).

In other embodiments of Method 2, or any of 2.1-2.53, wherein the fatty acid is a polyunsaturated fatty acid, the steps of Method 2 can be drawn as follows:
(a) converting the polyunsaturated fatty acid to a C₃₋₁₂ fatty acid ester, 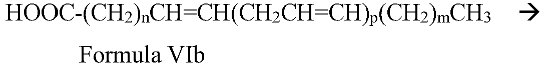 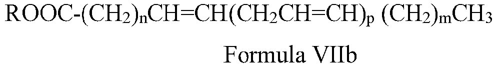 wherein n is an integer from 2 to 9 (e.g., 2, 3, 4, 5, 6, or 7) and m is an integer from 1 to 5 (e.g., 1 or 4), p is an integer from 1 to 7 (e.g., 1, 2, 3, 4, or 5), and R is a C₃₋₁₂ alkyl (e.g., isopropyl, sec-butyl, 2-ethylhexyl, isodecyl);
(b) performing ozonolysis with reductive work-up on the C₃₋₁₂ fatty acid ester to form the oxo-carboxylic acid ester and aldehyde by-products, 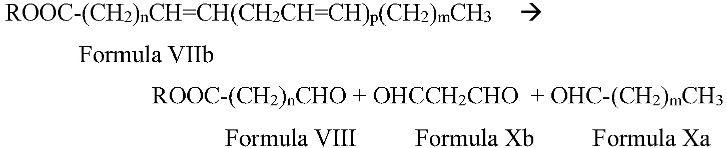 wherein n is an integer from 2 to 9 (e.g., 2, 3, 4, 5, 6, or 7) and m is an integer from 1 to 5 (e.g., 1 or 4), and R is a C₃₋₁₂ alkyl (e.g., isopropyl, sec-butyl, 2-ethylhexyl, isodecyl);
   and
(c) converting the oxo-carboxylic acid ester to the oxo-carboxylic acid product (e.g., by aqueous hydrolysis) 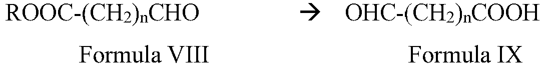 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl).

It is understood that the present methods embrace both cis and trans fatty acids as starting materials, although cis fatty acids are preferred because they are more widely dispersed in nature and available from common nuts, seeds, and vegetable oil precursors (e.g., macadamia nuts, guarana, olive oil, pecan oil, canola oil, jojoba oil, wallflower seed, mustard oil, flaxseed, sesame seed, chia seeds, walnuts, hemp seed oil, corn oil, peanut oil, borage oil, black currant oil, and safflower oil. Some of these cis-unsaturated fatty acids are also available from animal products, such as fish oils.

In some embodiments of Method 1, or any of 1.1-1.49, or Method 2, or any of 2.1-2.53, the present disclosure further provides a variation on said methods wherein the initial C₃₋₁₂ fatty acid ester is subjected to ozonolysis with reductive quenching to afford the oxo-carboxylic acid ester and aldehyde products, and after removal of the aldehyde, the oxo-carboxylic acid ester is then oxidized to the dicarboxylic acid ester, optionally followed by hydrolysis to the carboxylic acid product. For example, the present disclosure provides a method (Method 3) for preparing a high melting point dicarboxylic acid (e.g., brassylic acid) from a monounsaturated or polyunsaturated fatty acid (e.g., erucic acid), comprising the steps of:
(a) converting the fatty acid to a C₃₋₁₂ fatty acid ester;
(b) performing ozonolysis with reductive work-up on the C₃₋₁₂ fatty acid ester to form the oxo-carboxylic acid ester and aldehyde by-product;
(c) removing the aldehyde by-product;
(d) oxidizing the oxo-carboxylic acid ester to form the dicarboxylic acid ester; and
(e) converting the dicarboxylic acid ester to the dicarboxylic acid product (e.g., by aqueous hydrolysis);
   optionally, wherein the formation of ozonide intermediate of the ozonolysis step (b) is conducted in a flow reactor.

Thus, in particular embodiments of Method 3, the present disclosure provides a method for preparing a high melting point dicarboxylic acid (e.g., brassylic acid) from a monounsaturated or polyunsaturated fatty acid (e.g., erucic acid), comprising the steps of:
(a) converting the monounsaturated fatty acid to a C₃₋₁₂ fatty acid ester, 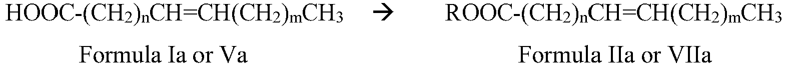 wherein n is an integer from 7 to 13 (e.g., 7, 9, 11, or 13) and m is an integer from 3 to 9 (e.g., 5 or 7), R is a C₃₋₁₂ alkyl (e.g., isopropyl, sec-butyl, 2-ethylhexyl, isodecyl);
(b) performing ozonolysis with reductive work-up on the C₃₋₁₂ fatty acid ester to form the oxo-carboxylic acid ester and aldehyde by-product, 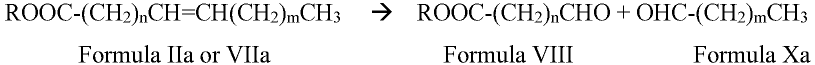 wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl);
(c) removing the aldehyde product of Formula Xa;
(d) oxidizing the oxo-carboxylic acid ester to form the dicarboxylic acid ester, 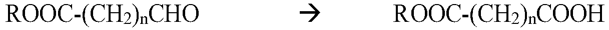 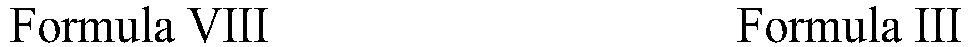 wherein n is an integer from 7 to 13 (e.g., 7, 9, 11, or 13), and R is a C₃₋₁₂ alkyl (e.g., isopropyl, sec-butyl, 2-ethylhexyl, isodecyl); and
(e) converting the dicarboxylic acid ester to the dicarboxylic acid product (e.g., by aqueous hydrolysis) 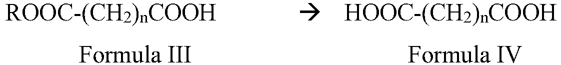
wherein n is an integer from 7 to 13 (e.g., 7, 9, 11, or 13) and m is an integer from 3 to 9 (e.g., 5 or 7), and R is a C₃₋₁₂ alkyl (e.g., isopropyl, sec-butyl, 2-ethylhexyl, isodecyl).

In all respects, the Method 3 may be carried as specifically described in any of the embodiments set forth hereinabove for Methods 1.1-1.49, or Methods 2.1-2.53, as appropriate.

Thus, in particular embodiments of Method 1, or any of 1.1-1.49, or Method 2, or any of 2.1-2.53, as modified above, or Method 3, wherein the fatty acid is erucic acid, the present disclosure provides a method of preparing brassylic acid from erucic acid, comprising the steps of:
(a) converting the erucic acid to a C₃₋₁₂ erucic acid ester, 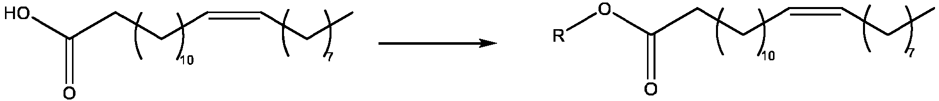 , wherein R is a C₃₋₁₂ alkyl (e.g., isopropyl, sec-butyl, 2-ethylhexyl, isodecyl);
(b) performing ozonolysis with reductive work-up on the C₃₋₁₂ erucic acid ester to form the brassyl aldehyde ester and nonanal by-product, 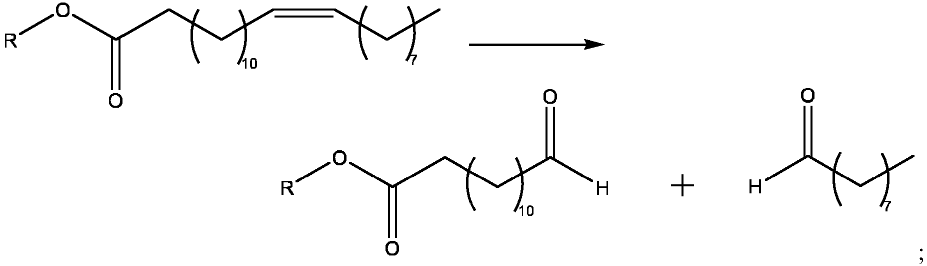
(c) removing the nonanal by-product (e.g., by distillation);
(d) oxidizing the brassyl aldehyde ester to brassylic acid ester (e.g., using vanadium pentoxide): 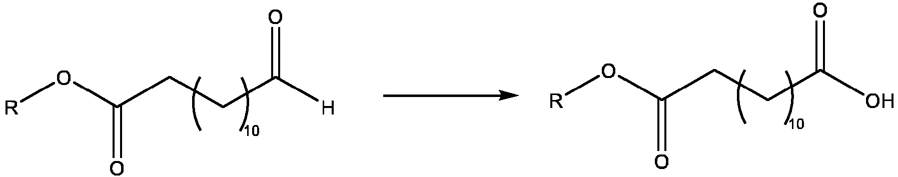
(e) converting the brassylic acid ester to brassylic acid (e.g., by aqueous hydrolysis)

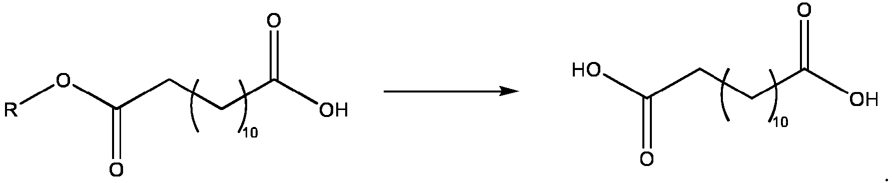

In addition, in any of the preceding embodiments, after the final hydrolysis step, the released C₃₋₁₂ alcohol (R-OH, wherein R is a C₃₋₁₂ alkyl (e.g., isopropyl, sec-butyl, 2-ethylhexyl, isodecyl)), may be recycled such that it is isolated from the hydrolysis step and used as a starting material for the initial ester formation step.

Thus, in one embodiment of the present disclosure, the following process is provided:

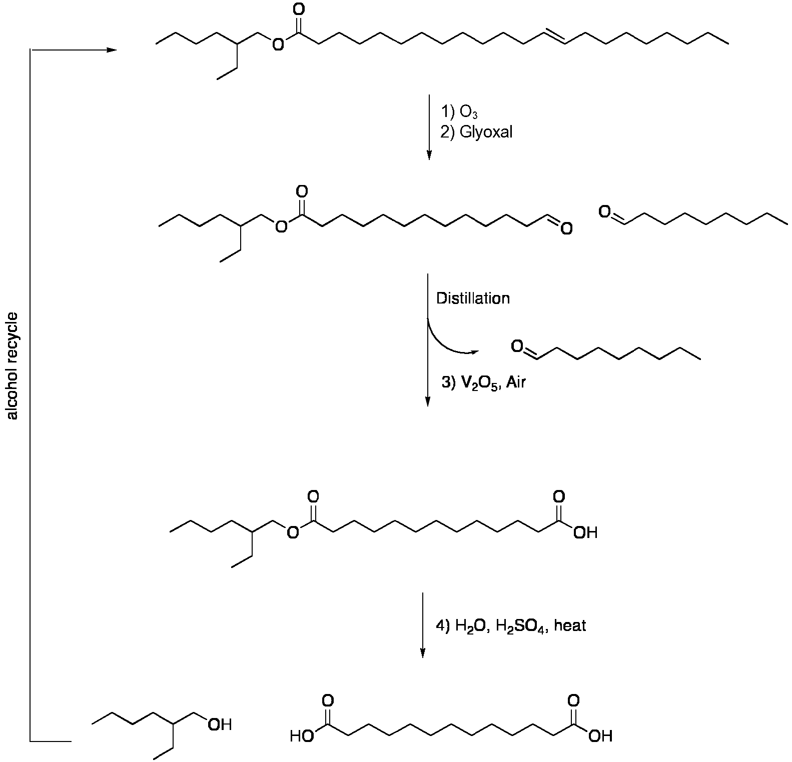

Initially, 2-ethylhexyl erucate is first prepared from 2-ethylhexanol (or, under some conditions, 2-ethyl-1-hexene) and erucic acid (not shown). Then, as shown above, the resulting ester is submitted to ozonolysis with reductive quenching using glyoxal, to afford 2-ethylhexyl brassyl aldehyde and nonanal. After removal of the nonanal, the 2-ethylhexyl brassyl aldehyde is oxidized to 2-ethylhexyl brassylate using vanadium pentoxide and air. The resulting ester is hydrolyzed using aqueous sulfuric acid to afford brassylic acid and 2-ethylhexanol, and the latter is recycled to the first step (ester formation).

It is understood that in the recitations of Method 1 and 1.1-1.49, and Method 2 and 2.1-2.53, and Method 3, where the singular terms "fatty acid ester," "dicarboxylic acid ester," "erucic acid ester," "brassylic acid ester," "oxo-carboxylic acid ester", and "brassyl aldehyde ester" are used, in some cases, for example where the ester is an isodecyl ester, the ester may be a mixture of structural isomeric esters. Thus, in such a case, the singular case "ester" recited in the above methods should be interpreted as referring to a mixture of isomeric esters.

As used herein, the term "dicarboxylic acid" refers to a linear alkyl dicarboxylic acid having two carboxylic acid groups at opposite ends of the linear alkane chain. Thus, it refers to a dicarboxylic acid of the formula HOOC-(CH₂)ₙCOOH. As used herein, the term "oxo-carboxylic acid" refers to a linear alkyl dicarbonyl compound wherein one end of the linear alkane chain ends in a carboxylic acid group and the other end of the linear alkane chain ends in an aldehyde group. Thus, it refers to a compound of the formula HOOC-(CH₂)ₙCHO. It is further understood that in chemical formulas used herein the group "CHO" or "OHC" refers to an aldehyde group, i.e., -C(=O)H.

As used herein, the term "fatty acid" refers to a saturated or unsaturated, monocarboxylic acid, generally having from 4 to 28 carbon atoms. However, it is also understood that, unless indicated otherwise, the term "fatty acid" refers herein to a monounsaturated or polyunsaturated fatty acid having 7 to 28 carbon atoms.

The term "high melting point dicarboxylic acid" or "high melting dicarboxylic acid" refers herein to a dicarboxylic acid, as defined herein, having a melting point of at least 60 °C. The term "high melting point oxo-carboxylic acid" or "high melting oxo-carboxylic acid" refers herein to an oxo-carboxylic acid, as defined herein, having a melting point of at least 50 °C.

The term "isodecyl" as used herein refers to any 10-carbon saturated alkyl chain that is not linear (i.e., not n-decyl). Examples of isodecyl groups include, but are not limited to, 2,4-dimethyloctan-2-yl, 2,6-dimethyl-octan-1-yl, 2,6-dimethyloctan-2-yl, 3,7-dimethyloctan-1-yl, and 3,7-dimethyloctan-3-yl.

It is understood that ozonolysis is a two-step process consisting of a first step in which an alkene is reacted with ozone to form a reactive ozonide intermediate (primary and secondary ozonides) and a second step which either oxidizes or reduces the ozonide mixture to provide carbonyl products. The terms "oxidative work-up" and "reductive work-up" refer to the conditions of this second step. It is understood that "oxidative work-up" exposes the reactive ozonides to an oxidizing reagent resulting in carboxylic acid products, while "reductive work-up" exposes the reactive ozonides to a reducing agent resulting in aldehyde products. It is further understood that either or both steps of the ozonolysis, unless provided otherwise herein, may be performed in a batch reactor or in flow reactor.

In some embodiments, traditional reducing agents are used for the "reductive work-up" of the ozonide intermediate mixture. Such agents include, but are not limited to sodium hydroxymethanesulfinate, hydroxymethanesulfinic acid, sulfite salts (e.g., sodium sulfite, sodium bisulfite, sodium metabisulfite, potassium sulfite, potassium bisulfite, potassium metabisulfite), sulfur dioxide, sodium dithionite, dimethyl sulfide, zinc, triphenylphosphine, thiourea, and formic acid, and hydrogenation methods over transition metal catalysts, such as palladium, platinum, and rhodium. However, in some embodiments, the novel ozonide reducing agent glyoxal is employed, optionally as an aqueous solution (e.g., 40% aqueous glyoxal).

### EXAMPLES

### Example 1 - Isodecyl Erucate

2,6-dimethyloct-2-ene (14 g, 0.1 mol) is combined with 16.7 g of erucic acid (0.05 mol) in a large glass vial, and the vial is then charged with 1.0 g of Amberlyst H+ catalyst. The reaction is then heated to 40 °C with stirring, melting the erucic acid and forming a single liquid phase. The reaction mixture is stirred for 3 days, until ¹H NMR and TLC show the desired product to have formed. The reaction mixture is then diluted with hexane, filtered through a pad of celite and silica to remove all catalyst, and it is then concentrated to remove hexane and residual 2,6-dimethyloct-2-ene. The resulting product, a mixture of 2,6-dimethyloctan-1-yl erucate and 2,6-dimethyl-octan-2-yl erucate, may be used crude in subsequent chemistry or further purified, e.g., via extraction, distillation, and/or chromatography.

### Example 2 - 2-Ethylhexyl Erucate

Erucic acid (338 g, 1 mol) is combined with 2-ethylhexanol (1.5 mol, 195 g). The mixture is then charged with 9.8 g of concentrated sulfuric acid (0.1 mol) and stirred at 60 °C under vacuum to remove water. After 6 hours of stirring, the reaction is removed from vacuum and heat, and neutralized with 0.1 mol of NaOH (6 M aqueous). The organic mixture is then washed with water and phase separated. The organic phase can then be taken on 'as-is', or can be further purified to remove any moisture and excess 2-ethylhexanol under vacuum distillation.

### Example 3 - Brassylic Acid

2-Ethylhexyl erucate (450 g, 1 mol) is contacted with 5% ozone in air in a temperature-controlled reactor at 35 °C until ozone consumption ceased and the reaction exotherm abated. 2M aqueous sodium hydroxide (1.5 equiv.) is then added to the reaction mixture slowly with stirring while maintaining the reaction temperature at 60 °C or below. Once the exotherm ceases, the reaction is stirred for an additional 2 hours at 60 °C to ensure completion of the quenching. The reaction is checked by DSC and iodometric titration to confirm quenching. The reaction is then neutralized with 2M aqueous sulfuric acid (1.5 equiv.). The phases are then separated and the organic phase is washed with water, then passed through a wiped film evaporator at reduced pressure to remove trace volatiles and to fraction off the nonanoic acid.

The heavy organic fraction containing the 2-ethylhexyl brassylate intermediate (and 2-ethylhexyl brassyl aldehyde) is then charged with 0.5 g vanadium pentoxide and the reaction is sparged with air at 50 °C for six hours. The mixture is then passed through a Celite filter to remove catalyst.

The filtrate is combined in a 1:1 v/v ratio with water, and then 5% by weight of concentrated sulfuric acid is added. The mixture is stirred vigorously with steam stripping to remove the 2-ethylhexanol as it forms. When the reaction is complete, it is cooled to room temperature and the aqueous phase is filtered to recover brassylic acid crystals.

### Example 5 - 2-Ethylhexyl Brassyl Aldehyde

2-Ethylhexyl erucate (450 g, 1 mol) is contacted with 5% ozone in air in a temperature-controlled reactor at 35 °C until ozone consumption ceased and the reaction exotherm abated. An aqueous solution of sodium sulfite (1.5 mol equivalents) is then added to the reaction mixture slowly with stirring while maintaining the reaction temperature at 60 °C or below. Once the exotherm ceases, the reaction is stirred for an additional 2 hours at 60 °C to ensure completion of the reductive quenching. The reaction is checked by DSC and iodometric titration to confirm quenching. The phases are then separated and the organic phase is washed with water, then passed through a wiped film evaporator at reduced pressure to remove trace volatiles and to fraction off the nonanal.

### Example 6 - 2-Ethylhexyl Brassyl Aldehyde via Glyoxal Reduction

2-Ethylhexyl erucate (450 g, 1 mol) is contacted with 5% ozone in air in a temperature-controlled reactor at 35 °C until ozone consumption ceased and the reaction exotherm abated. A 40% aqueous glyoxal solution (1.5 mol glyoxal) is then added to the reaction mixture slowly with stirring while maintaining the reaction temperature at 60 °C or below. Once the exotherm ceases, the reaction is stirred for an additional 2 hours at 60 °C to ensure completion of the reductive quenching. The reaction is checked by DSC and iodometric titration to confirm quenching. The phases are then separated and the organic phase is washed with water, then passed through a wiped film evaporator at reduced pressure to remove trace volatiles and to fraction off the nonanal, thus providing 2-ethylhexyl brassyl aldehyde in good yield and purity.

If desired, standard methods known to the artisan may be employed to convert the 2-ethylhexyl brassyl aldehyde to brassyl aldehyde. For example, a protection-hydrolysis-deprotection route, such as, forming an acetal, followed by base-catalyzed hydrolysis and deprotection of the acetal back to the aldehyde.

The Examples provided herein are exemplary only and are not intended to be limiting in any way to the various aspects and embodiments of the invention, which is defined by the claims.

## Claims

1. A method for preparing a high melting point dicarboxylic acid (e.g., brassylic acid) from a monounsaturated or polyunsaturated fatty acid (e.g., erucic acid), comprising the steps of:
(a) converting the fatty acid to a C₃₋₁₂ fatty acid ester;
(b) performing ozonolysis with oxidative work-up on the C₃₋₁₂ fatty acid ester to form a dicarboxylic acid ester and a carboxylic acid by-product;
and
(c) converting the dicarboxylic acid ester to the dicarboxylic acid product (e.g., by aqueous hydrolysis).

2. The method according to claim 1, wherein the fatty acid has a melting point above 15 °C, e.g., above 20 °C, or above 25 °C, or above 30 °C, e.g., up to 60 °C.

3. The method according to claim 1 or 2, wherein the dicarboxylic acid product has a melting point above 60 °C, e.g., above 70 °C, or above 80 °C, or above 90 °C, e.g., up to 60 °C.

4. The method according to claim 1, wherein the fatty acid is selected from erucic acid, palmitoleic acid, oleic acid, elaidic acid, vaccenic acid, gondonic acid, paullinic acid, nervonic acid, stearidonic acid, gamma-linolenic acid, eicosapentaenoic acid, arachidonic acid, docosatetraenonic acid, linolenic acid, linoleic acid, and linoleaidic acid; and optionally, wherein the product dicarboxylic acid is selected from brassylic acid, azelaic acid, adipic acid, glutaric acid, pimelic acid, undecanedioic acid, and pentaundecanedioic acid.

5. The method according to claim 1, wherein the dicarboxylic acid is brassylic acid, and the monounsaturated or polyunsaturated fatty acid is erucic acid, wherein the method comprises the steps of:
(a) converting erucic acid to a C₃₋₁₂ erucic acid ester, 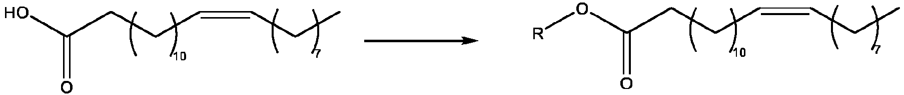 wherein R is a C₃₋₁₂ alkyl (e.g., isopropyl, sec-butyl, 2-ethylhexyl, isodecyl);
(b) performing ozonolysis with oxidative work-up on the C₃₋₁₂ erucic acid ester to form the corresponding brassylic acid ester and nonanoic acid, 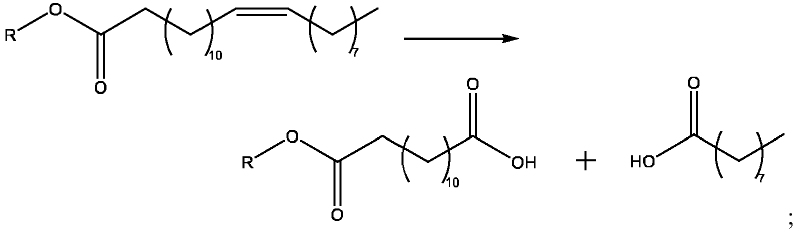 and
(c) converting the brassylic acid ester to brassylic acid (e.g., by aqueous hydrolysis)

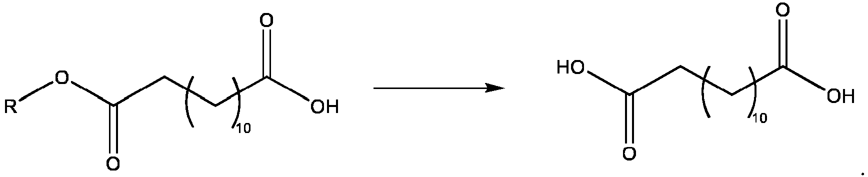

6. The method of claim 5, wherein R is C₃₋₁₀ alkyl (e.g., isopropyl, sec-butyl, 2-ethylhexyl, isodecyl).

7. The method of claim 5, wherein R is selected from isopropyl, n-butyl, sec-butyl, isobutyl, tert-butyl, n-pentyl, isopentyl, tert-amyl, n-hexyl, n-heptyl, 2-ethylheptyl, n-octyl, 2-ethylhexyl, n-nonyl, n-decyl, isodecyl (e.g., 2,4-dimethyloctan-2-yl, 2,6-dimethyl-octan-1-yl, 2,6-dimethyloctan-2-yl, 3,7-dimethyloctan-1-yl, and 3,7-dimethyloctan-3-yl).

8. The method of any one of claims 1-7, wherein the step (b) ozonolysis comprises (1) treating the fatty acid ester (e.g., erucic acid ester) with gaseous ozone in a carrier gas in a flow reactor, followed by (2) continuous quenching of the flow reactor product stream under oxidative conditions, optionally
wherein the step (b)(1) is carried out in a co-current flow reactor, e.g., a falling film reactor.

9. The method of claim 8, wherein the fatty acid ester (e.g., erucic acid ester) of step (a) is introduced into the reactor of step (b)(1) as an aqueous emulsion consisting of the neat fatty acid ester (e.g., erucic acid ester) and aqueous solution (e.g., aqueous buffer or water), for example, without any organic solvents.

10. The method of claim 8, wherein the fatty acid ester (e.g., erucic acid ester) of step (a) is introduced into the reactor of step (b)(1) as the neat fatty acid ester, for example, without any organic solvents or water.

11. The method of any one of claims 8-10, wherein step (b)(2) comprises continuously mixing the output stream from the reactor of step (b)(1) with a quenching solution, e.g., an aqueous solution, organic solution, or aqueous/organic emulsion, comprising an oxidizing agent.

12. The method of any one of claims 1-11, wherein step (a) is carried out by reacting the fatty acid (e.g., erucic acid) with the corresponding C₃₋₁₂ alcohol or C₃₋₁₂ alkene, in the presence of an acidic catalyst (e.g., a solid acidic resin).

13. The method of any one of claims 1-12, wherein the product stream from step (b) consists essentially of the dicarboxylic acid ester, the carboxylic acid by-product, and any unreacted fatty acid ester, or carried over fatty acid, for example, wherein the product stream from step (b) consists essentially of brassylic ester, nonanoic acid, and any unreacted erucic acid ester, or carried over unreacted erucic acid; and optionally wherein the product stream from step (b) and/or step (c) does not contain any reduction products, e.g., the product stream does not contain any aldehydes, alcohols, aldehyde/esters, aldehyde/acids, alcohol/esters, or alcohol acids, such as nonanal, nonanol, 13-oxotridecanoic acid, 13-oxotridecanoate esters, 13-hydroxytridecanoic acid, or 13-hydroxytridecanoate esters.

14. A method for preparing a high melting point oxo-carboxylic acid (e.g., brassyl aldehyde) from a monounsaturated or polyunsaturated fatty acid (e.g., erucic acid), comprising the steps of:
(a) converting the fatty acid to a C₃₋₁₂ fatty acid ester;
(b) performing ozonolysis with reductive work-up on the C₃₋₁₂ fatty acid ester to form an oxo-carboxylic acid ester and an aldehyde by-product;
and
(c) converting the oxo-carboxylic acid ester to the oxo-carboxylic acid product (e.g., by aqueous hydrolysis);
wherein the formation of ozonide intermediate of the ozonolysis step (b) is conducted in a flow reactor, optionally
wherein the method further comprises the step of isolating the aldehyde by-product (e.g., nonanal) from the crude product, or partially purified product of step (b) or from the crude product, or partially purified product of step (c).

15. A method for preparing a high melting point dicarboxylic acid (e.g., brassylic acid) from a monounsaturated or polyunsaturated fatty acid (e.g., erucic acid), comprising the steps of:
(a) converting the fatty acid to a C₃₋₁₂ fatty acid ester;
(b) performing ozonolysis with reductive work-up on the C₃₋₁₂ fatty acid ester to form an oxo-carboxylic acid ester and an aldehyde by-product;
(c) removing the aldehyde by-product;
(d) oxidizing the oxo-carboxylic acid ester to form a dicarboxylic acid ester; and
(e) converting the dicarboxylic acid ester to the dicarboxylic acid product (e.g., by aqueous hydrolysis);
optionally, wherein the formation of ozonide intermediate of the ozonolysis step (b) is conducted in a flow reactor.

## Patentansprüche

1. Verfahren zum Herstellen einer Dicarbonsäure mit hohem Schmelzpunkt (z. B. Brassylsäure) aus einer monoungesättigten oder polyungesättigten Fettsäure (z. B. Erucasäure), umfassend die folgenden Schritte:
(a) Umwandeln der Fettsäure in einen C₃₋₁₂-Fettsäureester;
(b) Durchführen von Ozonolyse mit oxidativer Aufarbeitung an dem C₃₋₁₂-Fettsäureester, um einen Dicarbonsäureester und ein Carbonsäure-Nebenprodukt zu bilden;
und
(c) Umwandeln des Dicarbonsäureesters in das Dicarbonsäureprodukt (z. B. durch wässrige Hydrolyse).

2. Verfahren nach Anspruch 1, wobei die Fettsäure einen Schmelzpunkt über 15 °C, z. B. über 20 °C oder über 25 °C oder über 30 °C, z. B. bis zu 60 °C aufweist.

3. Verfahren nach Anspruch 1 oder 2, wobei das Dicarbonsäureprodukt einen Schmelzpunkt über 60 °C, z. B. über 70 °C oder über 80 °C oder über 90 °C, z. B. bis zu 60 °C aufweist.

4. Verfahren nach Anspruch 1, wobei die Fettsäure ausgewählt ist aus Erucasäure, Palmitoleinsäure, Ölsäure, Elaidinsäure, Vaccensäure, Gondonsäure, Paullinsäure, Nervonsäure, Stearidonsäure, Gamma-Linolensäure, Eicosapentaensäure, Arachidonsäure, Docosatetraenonsäure, Linolensäure, Linolsäure und Linoleaidinsäure; und wobei die Produktdicarbonsäure optional ausgewählt ist aus Brassylsäure, Azelainsäure, Adipinsäure, Glutarsäure, Pimelinsäure, Undecandisäure und Pentaundecandisäure.

5. Verfahren nach Anspruch 1, wobei die Dicarbonsäure Brassylsäure ist und die monoungesättigte oder polyungesättigte Fettsäure Erucasäure ist, wobei das Verfahren die folgenden Schritte umfasst:
(a) Umwandeln von Erucasäure in einen C₃₋₁₂-Erucasäureester, 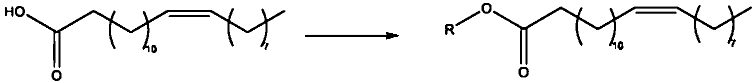 wobei R ein C₃₋₁₂-Alkyl (z. B. Isopropyl, sec-Butyl, 2-Ethylhexyl, Isodecyl) ist;
(b) Durchführen von Ozonolyse mit oxidativer Aufarbeitung an dem C₃₋₁₂-Erucasäureester, um den entsprechenden Brassylsäureester und Nonansäure zu bilden, 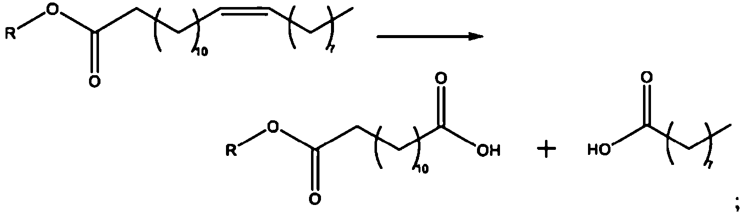 und
(c) Umwandeln des Brassylsäureesters in Brassylsäure (z. B. durch wässrige Hydrolyse).

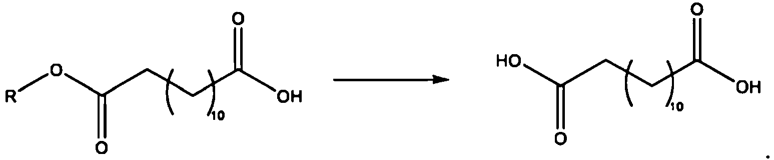

6. Verfahren nach Anspruch 5, wobei R ein C₃₋₁₀-Alkyl (z. B. Isopropyl, sec-Butyl, 2-Ethylhexyl, Isodecyl) ist.

7. Verfahren nach Anspruch 5, wobei R ausgewählt ist aus Isopropyl, n-Butyl, sec-Butyl, Isobutyl, tert-Butyl, n-Pentyl, Isopentyl, tert-Amyl, n-Hexyl, n-Heptyl, 2-Ethylheptyl, n-Octyl, 2-Ethylhexyl, n-Nonyl, n-Decyl, Isodecyl (z. B. 2,4-Dimethyloctan-2-yl, 2,6-Dimethyloctan-1-yl, 2,6-Dimethyloctan-2-yl, 3,7-Dimethyloctan-1-yl und 3,7-Dimethyloctan-3-yl).

8. Verfahren nach einem der Ansprüche 1-7, wobei der Schritt (b) Ozonolyse (1) Behandeln des Fettsäureesters (z. B. Erucasäureester) mit gasförmigem Ozon in einem Trägergas in einem Strömungsreaktor, gefolgt von (2) kontinuierlichem Quenchen des Strömungsreaktor-Produktstroms unter oxidativen Bedingungen umfasst, wobei
der Schritt (b)(1) optional in einem Gleichstromreaktor, z. B. einem Fallfilmreaktor, ausgeführt wird.

9. Verfahren nach Anspruch 8, wobei der Fettsäureester (z. B. Erucasäureester) aus Schritt (a) als eine wässrige Emulsion, bestehend aus dem reinen Fettsäureester (z. B. Erucasäureester) und einer wässrigen Lösung (z. B. wässrigem Puffer oder Wasser), beispielsweise ohne organische Lösungsmittel, in den Reaktor von Schritt (b)(1) eingeführt wird.

10. Verfahren nach Anspruch 8, wobei der Fettsäureester (z. B. Erucasäureester) aus Schritt (a) als der reine Fettsäureester, beispielsweise ohne organische Lösungsmittel oder Wasser, in den Reaktor von Schritt (b)(1) eingeführt wird.

11. Verfahren nach einem der Ansprüche 8-10, wobei Schritt (b)(2) kontinuierliches Mischen des Ausgabestroms aus dem Reaktor von Schritt (b)(1) mit einer Quenchlösung, z. B. einer wässrigen Lösung, einer organischen Lösung oder einer wässrig-organischen Emulsion umfasst, die ein Oxidationsmittel umfasst.

12. Verfahren nach einem der Ansprüche 1-11, wobei Schritt (a) durch Umsetzen der Fettsäure (z. B. Erucasäure) mit dem entsprechenden C₃₋₁₂-Alkohol oder C₃₋₁₂-Alken in Gegenwart eines sauren Katalysators (z. B. eines festen sauren Harzes) ausgeführt wird.

13. Verfahren nach einem der Ansprüche 1-12, wobei der Produktstrom aus Schritt (b) im Wesentlichen aus dem Dicarbonsäureester, dem Carbonsäure-Nebenprodukt und jeglichem nicht umgesetzten Fettsäureester oder einer übertragenen Fettsäure besteht, wobei der Produktstrom beispielsweise aus Schritt (b) im Wesentlichen aus Brassylsäureester, Nonansäure und jeglichem nicht umgesetzten Erucasäureester oder einer übertragenen nicht umgesetzten Erucasäure besteht; und wobei der Produktstrom aus Schritt (b) und/oder Schritt (c) keine Reduktionsprodukte enthält, z. B. enthält der Produktstrom optional keine Aldehyde, Alkohole, Aldehyde/Ester, Adehyde/Säuren, Alkohol/Ester, oder Alkoholsäuren wie Nonanal, Nonanol, 13-Oxotridecansäure, 13-Oxotridecanoatester, 13-Hydroxytridecansäure oder 13-Hydroxytridecanoatester.

14. Verfahren zum Herstellen einer Oxocarbonsäure mit hohem Schmelzpunkt (z. B. Brassylaldehyd) aus einer monoungesättigten oder polyungesättigten Fettsäure (z. B. Erucasäure), umfassend die folgenden Schritte:
(a) Umwandeln der Fettsäure in einen C₃₋₁₂-Fettsäureester;
(b) Durchführen von Ozonolyse mit reduktiver Aufarbeitung an dem C₃₋₁₂-Fettsäureester, um einen Oxocarbonsäureester und ein Aldehyd-Nebenprodukt zu bilden;
und
(c) Umwandeln des Oxocarbonsäureesters in das Oxocarbonsäureprodukt (z. B. durch wässrige Hydrolyse);
wobei die Bildung des Ozonid-Zwischenprodukts des Ozonolyseschritts (b) in einem Strömungsreaktor ausgeführt wird,
wobei das Verfahren optional weiter den Schritt des Isolierens des Aldehyd-Nebenprodukts (z. B. Nonanal) aus dem Rohprodukt oder dem teilweise gereinigten Produkt aus Schritt (b) oder aus dem Rohprodukt oder dem teilweise gereinigten Produkt aus Schritt (c) umfasst.

15. Verfahren zum Herstellen einer Dicarbonsäure mit hohem Schmelzpunkt (z. B. Brassylsäure) aus einer monoungesättigten oder polyungesättigten Fettsäure (z. B. Erucasäure), umfassend die folgenden Schritte:
(a) Umwandeln der Fettsäure in einen C₃₋₁₂-Fettsäureester;
(b) Durchführen von Ozonolyse mit reduktiver Aufarbeitung an dem C₃₋₁₂-Fettsäureester, um einen Oxocarbonsäureester und ein Aldehyd-Nebenprodukt zu bilden;
(c) Entfernen des Aldehyd-Nebenprodukts;
(d) Oxidieren des Oxocarbonsäureesters, um einen Dicarbonsäureester zu bilden; und
(e) Umwandeln des Dicarbonsäureesters in das Dicarbonsäureprodukt (z. B. durch wässrige Hydrolyse);
wobei die Bildung des Ozonid-Zwischenprodukts des Ozonolyseschritts (b) optional in einem Strömungsreaktor ausgeführt wird.

## Revendications

1. Procédé de préparation d'un acide dicarboxylique à point de fusion élevé (par exemple, l'acide brassylique) à partir d'un acide gras monoinsaturé ou polyinsaturé (par exemple, l'acide érucique), comprenant les étapes de :
(a) conversion de l'acide gras en un ester d'acide gras en C₃₋₁₂ ;
(b) réalisation d'une ozonolyse avec traitement oxydatif sur l'ester d'acide gras en C₃₋₁₂ pour former un ester d'acide dicarboxylique et un sous-produit acide carboxylique ;
et
(c) conversion de l'ester d'acide dicarboxylique en produit acide dicarboxylique (par exemple, par hydrolyse aqueuse).

2. Procédé selon la revendication 1, dans lequel l'acide gras présente un point de fusion supérieur à 15 °C, par exemple supérieur à 20 °C, ou supérieur à 25 °C, ou supérieur à 30 °C, par exemple allant jusqu'à 60 °C.

3. Procédé selon la revendication 1 ou 2, dans lequel le produit acide dicarboxylique présente un point de fusion supérieur à 60 °C, par exemple supérieur à 70 °C, ou supérieur à 80 °C, ou supérieur à 90 °C, par exemple allant jusqu'à 60 °C.

4. Procédé selon la revendication 1, dans lequel l'acide gras est sélectionné parmi l'acide érucique, l'acide palmitoléique, l'acide oléique, l'acide élaïdique, l'acide vaccénique, l'acide gondonique, l'acide paullinique, l'acide nervonique, l'acide stéaridonique, l'acide gamma-linolénique, l'acide eicosapentaénoïque, l'acide arachidonique, l'acide docosatétraénonique, l'acide linolénique, l'acide linoléique et l'acide linoléaïdique ; et, éventuellement, dans lequel le produit acide dicarboxylique est sélectionné parmi l'acide brassylique, l'acide azélaïque, l'acide adipique, l'acide glutarique, l'acide pimélique, l'acide undécanedioïque et l'acide pentaundécanedioïque.

5. Procédé selon la revendication 1, dans lequel l'acide dicarboxylique est l'acide brassylique et l'acide gras monoinsaturé ou polyinsaturé est l'acide érucique, dans lequel le procédé comprend les étapes de :
(a) conversion de l'acide érucique en un ester d'acide érucique en C₃₋₁₂, 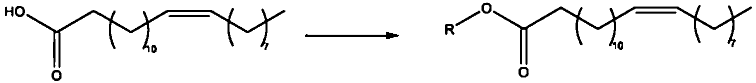 dans lequel R est alkyle en C₃₋₁₂ (par exemple, isopropyle, sec-butyle, 2-éthylhexyle, isodécyle) ;
(b) réalisation d'une ozonolyse avec traitement oxydatif sur l'ester d'acide érucique en C₃₋₁₂ pour former l'ester d'acide brassylique et l'acide nonanoïque correspondants, 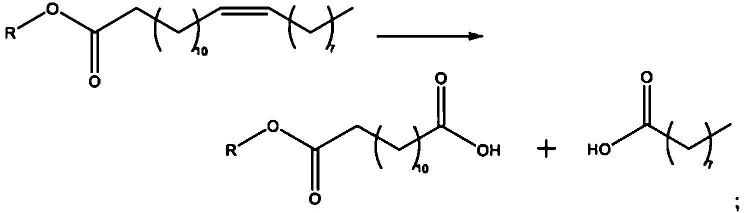 et
(c) conversion de l'ester d'acide brassylique en acide brassylique (par exemple, par hydrolyse aqueuse)

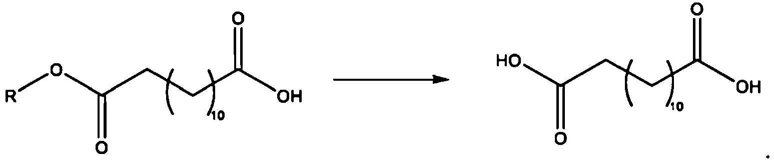

6. Procédé selon la revendication 5, dans lequel R est alkyle en C₃₋₁₀ (par exemple, isopropyle, sec-butyle, 2-éthylhexyle, isodécyle).

7. Procédé selon la revendication 5, dans lequel R est sélectionné parmi l'isopropyle, le n-butyle, le sec-butyle, l'isobutyle, le tert-butyle, le n-pentyle, l'isopentyle, le tert-amyle, le n-hexyle, le n-heptyle, le 2-éthylheptyle, le n-octyle, le 2-éthylhexyle, le n-nonyle, le n-décyle, l'isodécyle (par exemple, le 2,4-diméthyloctan-2-yle, le 2,6-diméthyl-octan-1-yle, le 2,6-diméthyloctan-2-yle, le 3,7-diméthyloctan-1-yle et le 3,7-diméthyloctan-3-yle).

8. Procédé selon l'une quelconque des revendications 1-7, dans lequel l'étape (b) d'ozonolyse comprend (1) le traitement de l'ester d'acide gras (par exemple, l'ester d'acide érucique) avec de l'ozone gazeux dans un gaz vecteur au sein d'un réacteur continu, suivi de (2) la désactivation continue du flux de produit du réacteur continu dans des conditions oxydantes, éventuellement
dans lequel l'étape (b)(1) est réalisée dans un réacteur continu à cocourant, par exemple un réacteur à film tombant.

9. Procédé selon la revendication 8, dans lequel l'ester d'acide gras (par exemple, l'ester d'acide érucique) de l'étape (a) est introduit dans le réacteur de l'étape (b)(1) sous forme d'émulsion aqueuse consistant en l'ester d'acide gras pur (par exemple, l'ester d'acide érucique) et de solution aqueuse (par exemple, un tampon aqueux ou de l'eau), par exemple, sans aucun solvant organique.

10. Procédé selon la revendication 8, dans lequel l'ester d'acide gras (par exemple, l'ester d'acide érucique) de l'étape (a) est introduit dans le réacteur de l'étape (b)(1) sous forme d'ester d'acide gras pur, par exemple, sans aucun solvant organique ni eau.

11. Procédé selon l'une quelconque des revendications 8-10, dans lequel l'étape (b)(2) comprend le mélange continu du flux de sortie du réacteur de l'étape (b)(1) avec une solution de désactivation, par exemple une solution aqueuse, une solution organique ou une émulsion aqueuse/organique, comprenant un agent oxydant.

12. Procédé selon l'une quelconque des revendications 1-11, dans lequel l'étape (a) est réalisée en faisant réagir l'acide gras (par exemple, l'acide érucique) avec l'alcool en C₃₋₁₂ ou l'alcène en C₃₋₁₂ correspondant, en présence d'un catalyseur acide (par exemple, une résine acide solide).

13. Procédé selon l'une quelconque des revendications 1-12, dans lequel le flux de produit de l'étape (b) consiste essentiellement en l'ester d'acide dicarboxylique, le sous-produit acide carboxylique et tout ester d'acide gras n'ayant pas réagi, ou l'acide gras transféré; par exemple, dans lequel le flux de produit de l'étape (b) consiste essentiellement en l'ester brassylique, l'acide nonanoïque et tout ester d'acide érucique n'ayant pas réagi, ou l'acide érucique transféré n'ayant pas réagi ; et, éventuellement, dans lequel le flux de produit de l'étape (b) et/ou de l'étape (c) ne contient aucun produit de réduction, par exemple, le flux de produit ne contient aucun aldéhyde, alcool, aldéhyde/ester, aldéhyde/acide, alcool/ester, ni acide alcoolique, tel que le nonanal, le nonanol, l'acide 13-oxotridécanoïque, les esters 13-oxotridécanoate, l'acide 13-hydroxytridécanoïque ou les esters 13-hydroxytridécanoate.

14. Procédé de préparation d'un acide oxo-carboxylique à point de fusion élevé (par exemple, l'aldéhyde brassylique) à partir d'un acide gras monoinsaturé ou polyinsaturé (par exemple, l'acide érucique), comprenant les étapes de :
(a) conversion de l'acide gras en un ester d'acide gras en C₃₋₁₂ ;
(b) réalisation d'une ozonolyse avec traitement réducteur sur l'ester d'acide gras en C₃₋₁₂ pour former un ester d'acide oxo-carboxylique et un sous-produit aldéhyde ;
et
(c) conversion de l'ester d'acide oxo-carboxylique en produit acide oxo-carboxylique (par exemple, par hydrolyse aqueuse) ;
dans lequel la formation de l'intermédiaire ozonide de l'étape d'ozonolyse (b) est réalisée dans un réacteur continu, éventuellement
dans lequel le procédé comprend en outre l'étape d'isolement du sous-produit aldéhyde (par exemple, le nonanal) du produit brut ou du produit partiellement purifié de l'étape (b) ou du produit brut ou du produit partiellement purifié de l'étape (c).

15. Procédé de préparation d'un acide dicarboxylique à point de fusion élevé (par exemple, l'acide brassylique) à partir d'un acide gras monoinsaturé ou polyinsaturé (par exemple, l'acide érucique), comprenant les étapes de :
(a) conversion de l'acide gras en un ester d'acide gras en C₃₋₁₂ ;
(b) réalisation d'une ozonolyse avec traitement réducteur sur l'ester d'acide gras en C₃₋₁₂ pour former un ester d'acide oxo-carboxylique et un sous-produit aldéhyde ;
(c) élimination du sous-produit aldéhyde ;
(d) oxydation de l'ester d'acide oxo-carboxylique pour former un ester d'acide dicarboxylique ; et
(e) conversion de l'ester d'acide dicarboxylique en produit acide dicarboxylique (par exemple, par hydrolyse aqueuse) ;
éventuellement, dans lequel la formation de l'intermédiaire ozonide de l'étape d'ozonolyse (b) est réalisée dans un réacteur continu.
